# EUROPEAN PATENT SPECIFICATION

(11) **EP 0 876 142 B1**
(45) Date of publication and mention of the grant of the patent: **26.09.2001**
(21) Application number: 96903489.1
(22) Date of filing: 16.01.1996
(51) Int. Cl.: A61K 9/22, A61K 9/52

(54) **TOPICAL DELIVERY OF DRUGS TO THE LOWER GASTROINTESTINAL TRACT**
ÖRTLICHE ARZNEISTOFFABGABE IN TIEFEREN MAGENDARMTRAKTABSCHNITTEN
ADMINISTRATION TOPIQUE DE MEDICAMENTS DANS LE TRACTUS GASTRO-INTESTINAL INFERIEUR

(43) Date of publication of application: 11.11.1998
(73) Proprietor: Advanced Polymer Systems, Inc., Redwood City, CA 94063 (US)
(72) Inventor: BERLINER, David, L., Atherton, CA 94025 (US); NACHT, Sergio, Redwood City, CA 94061 (US)
(74) Representative: Harrison, David Christopher
(86) International application number: US9600512
(87) International publication number: WO9725980

(56) References cited:
- WO-A-91/16881
- WO-A-91/19483
- DD-A- 291 668

## Description

This invention relates to the treatment of diseases of the colon, such as inflammatory bowel disease. More particularly, it relates to a dosage form for an active agent and the method of its use in topically treating disease in the colon.

Many conditions either originate or are expressed in the lumen or tissue intermediate to the lumen of the gastrointestinal (G.I.) tract. One group of such conditions to which the present invention is directed are inflammatory bowel diseases such as ulcerative colitis and Crohn's disease. Current therapy methods for inflammatory bowel diseases usually use a formulation from which the drug is absorbed systemically even though the preferred site of action may be at or near the site of absorption. A relatively high systemic concentration is necessary in order to ensure an efficacy in local concentration of the drug. An example of this is the administration of prednisolone for inflammatory bowel disease. The steroid is given to elicit a local action but is absorbed systemically, which if continued for prolonged exposure may result in atrophy of adrenal glands or cause other side effects systemically.

Prodrug techniques have been used to prevent systemic absorption. This method requires a prodrug which is not absorbed and which undergoes a transformation to give the active species after passing the G.I. absorption window or zone for the active species. An example of this is the use of sulpha solazine for inflammatory bowel disease.

While the prodrug approach has worked for some drugs it is limited in scope due to its dependence upon the chemistry of the drug, prodrug, and G.I. environment. A new prodrug must be developed, if possible, for each active species.

The present invention circumvents the above problems. There is described a method for treating certain diseases of the colon comprising (a) providing a dosage form containing a plurality of rigid cross-linked polymer beads, each defining a substantially noncollapsible internal pore network, and a therapeutically effective amount of an active agent in said pore network selected from the group consisting of corticosteroids and non-steroidal anti-inflammatory agents for treatment of inflammatory bowel disease, anti-tumor agents for treatment of colonic malignancies, anti-parasitic agents for treatment of parasites, and antibiotics for treatment of infections, said dosage form being treated to initially remain intact in the gastrointestinal tract, the treatment degrading in or near the large intestine whereby active agent is there released at a slow controlled rate; and (b) orally ingesting said dosage form. According to the present invention there is provided (copy claim 1).

The compositions used in the present method have been used as a delivery system for external topical skin administration and in such an environment have been shown to be capable of releasing an active substance at a controlled rate. It has now been found that such delivery systems utilizing the particular active agents herein described can be safely and efficaciously utilized internally of the G.I. tract and delivered to the desired location where the diseased tissue resides. Because the delivery systems release the active substances at a slow controlled rate systemic absorption is slowed or essentially prevented while at the same time a sufficiently high local concentration of the drug is provided to be effective in treating the disease. To the extent that some systemic absorption occurs with the corticosteroids, there is substantially no adverse side effects because of the slow rate of systemic absorption and because the particular corticosteroids employed in this invention are either not adversely reactive in the body or are so rapidly metabolized in the body as to have no significant adverse impact.

In one preferred embodiment the dosage form is a pharmaceutical capsule or tablet containing the polymer beads with the selected active agent in their porous network. The active agent, such as a corticosteroid, will generally be present in each pharmaceutical capsule or tablet in the amount of 1-100 mg., frequently in the amount of about 5-20 mg.

Preferred polymer beads are formed from a copolymer selected from styrene-divinylbenzene and methyl methacrylate-ethylene glycol dimethacrylate, and have a diameter of about 5-200 microns, preferably about 10-40 microns.

The preferred embodiment also provides for control of the release zone of active ingredients at the desired site of action. For example, only the large intestine (a portion or its entire length) is affected in ulcerative colitis, whereas in Crohn's disease both the terminal ileum and the ascending colon are affected. In the preferred embodiment the selected dosage form is treated so that it will pass through preliminary stages of the G.I. tract and release active agent at the affected location. In the case of ulcerative colitis the dosage form is treated to initially remain intact in the G.I. tract and to degrade in or near the large intestine. Similarly, in Crohn's disease the dosage form is treated to initially remain intact in the G.I. tract until reaching the junction of the ileum with the colon and then degrades so as to release active agent in that location. This is accomplished by treating bolt the polymer beads and the dosage form .

For example, in one aspect of the preferred embodiment for treating Crohn's disease the polymer beads in the dosage form are coated with pectin. The corticosteroid is not released until enzymes normally present in the colon react with and remove the pectin so that entrapped corticosteroid is then released.

### BRIEF DESCRIPTION OF THE DRAWINGS

Figure 1 plots the *in vitro* release of hydrocortisone from porous polymeric particles.
Figure 2 shows daily fecal dry weight of the three groups of rats used in the experiment of Example 4.
Figure 3 is a measurement of radioactivity in feces of the rats observed in the experiment of Example 4.

### DESCRIPTION OF THE PREFERRED EMBODIMENT

The beads or microspheres used in connection with the present invention are known in the art and are described in detail in US-A- 5,145,675.

In particular, the present invention contemplates the use of co-polymers of styrene and divinylbenzene, the synthesis of which is disclosed in the above-referenced '675 patent in Example 1.1. The same example also illustrates a method for entrapping steroids within the porous network of the polymeric beads. Similarly, the present invention in the preferred embodiment contemplates the use of copolymers of methyl methacrylate and ethylene glycol dimethacrylate. Preparation of such co-polymer particles is described in the above referenced '675 patent in Example 6.2. Particles of the aforementioned types are commercially available from Advanced Polymer Systems of Redwood City, California, in the form of empty particles or as particles which have been loaded with the active agents utilized in the present invention.

As mentioned above, the polymeric particles containing the selected corticosteroid or other active agent may be treated to control the point in the G.I. tract that release of the active agent commences. In this regard, the dosage form can be coated with enteric blocking agents which remain intact in the stomach where they are gastro resistant but which degrade in the intestines and are enterosoluble. Blocking agents suitable for use in the present invention are disclosed in US-A-5,316,774 for "Blocked Polymeric Particles Having Internal Pore Networks for Delivering Active Substances to Selected Environments."

One class of such materials suitable as enteric blocking agents are those which remain intact in the environment of the stomach but solubilize at the higher pH of the intestines. Materials of this type are known in the art where they have been used as coating for solid core drug formulations. The most effective enteric materials are polyacids having a pKₐ of from about 3 to 5. Exemplary materials include fat-fatty acid mixtures, ethyl cellulose, cellulose acetate phthalates, and the like.

Also suitable as enteric coatings are various poly(meth)acrylates which may be introduced to the polymeric carrier particles or dosage form either by in *situ* polymerization or by absorption of an aqueous dispersion of the materials. Suitable poly(meth)acrylates include copolymers of methylmethacrylate and ethylacrylate as ester components with methacrylic acid which contain carboxylic groups that are transformed to carboxylate groups at a pH of from about 5 to 7. They are thus able to form water-insoluble materials which are resistant to gastric juices and methacrylate ester copolymers which are insoluble over the entire physiological pH range. Specific copolymers useful as enteric materials are as follows.

| Enteric Material | Molecular Weight | Preferred Monomer Ratio |
|---|---|---|
| poly (methacrylic acid, ethylacrylate) copolymer | 250 kD | 1:1 |
| poly (methacrylic acid, methylmethacrylate) copolymer | 135 kD | 1:2 to 1:2 |
| poly (ethylacrylate, methacrylate) trimethyl-ammoniaethylmethacrylate chloride | 150 kD | 1:2:0:2 |
| poly (ethylacrylate, methylmethacrylate) trimethylammoniaethyl-methacrylate chloride | 150 kD | 1:2:0:2 |

The present invention utilizes any suitable dosage form that can be used for oral drug delivery. In this regard the polymeric particles carrying the drug may be incorporated into a variety of known dosage forms, as described in, for example, Remington's Pharmaceutical Sciences, Mack Publishing Company, Easton Pennsylvania, 16th Ed., 1982, the disclosure of which is incorporated herein by reference. The composition or formulation to be administered will contain a preselected quantity of the active substances(s) contained within the polymeric particles which are dispersed therein. Usually, a pharmaceutically acceptable non-toxic dosage form is prepared using conventional excipients, such as pharmaceutical grades of mannitol, lactose, starch, magnesium stearate, sodium saccharin, talcum, cellulose, glucose, sucrose, magnesium, carbonate, and the like. Such compositions may be in the form of solutions, suspensions, tablets, pills, capsules, powders, and the like.

As noted above, the invention is directed to the treatment of four different types of diseases of the colon. In the preferred embodiment the dosage form and method is particularly suited for the treatment of inflammatory bowel disease. Preferred drugs utilized in the dosage form for treatment of inflammatory bowel disease include hydrocortisone, beclomethasone dipropionate, tixocortol pivalate, budesonide, dexamethasone, prednisone, prednisolone and triamcinolone acetonide. In addition to the preferred corticosteroids, non-steroidal anti-inflammatory agents are also contemplated, such as amino salicylate and sulfasalazione. In addition, other agents which have been found to beneficially treat anti-inflammatory bowel disease may be delivered by the present composition and method. For example, recent clinical studies have shown that ulcerative colitis may be beneficially treated with cyclosporine, a drug that has usually been given to transplant patients. Another class of drugs that is contemplated is referred to as prodrugs wherein they are entrapped in the polymeric particles in the same manner as the preferred drugs previously mentioned. Examples of prodrugs are dexamethasone-succinate-dextran (Gastroenterology, 1994, 106:2 405-413), budesonide-β-D-glucuronide (Gut, 1994, 35: 1439-1446), and dexamethasone-β-D-glucuronide (Pharm Res, 1993, 10: 1553-1562).

With respect to the treatment of colonic malignancies, a suitable anti-tumor agent which is known in the art for the treatment of localized malignancies will be incorporated in the dosage form. Examples of anti-tumor agents suitable for use in this invention are methotrexate, 5-fluorouracil, and similarly functioning anti-neoplastic agents, such as tamoxifen, cyclophosphamide, mercaptopurine etoposide, indomethacin, semustine, fluorouracil, floxuridine and mitomycin. For the treatment of infections of the colon, antibiotics (including antibacterials) which are suitable for use in this invention include sulphanilamides and their derivatives, and other antibiotics specifically designed to treat particular bacterial infections associated with food ingestion. Additional examples include sulfonamides, norfloxacin, chloramphenicol, tetracyclines and vancomycin.

For treatment of parasites, suitable anti-parasitic agents include diloxanide furoate, metronidazole, quinacrine, tetracyclines, iodoquinol, dehydroemetine, amphotericin B, mebendazole and thiabendazole.

As noted, the key to obtaining release of the drug from the dosage form in or near the colon lies in the use of a coating that is not breached or significantly removed until reaching the colon where bacteria that are specific to and generally confined in the colon exist that attack and solubilize or otherwise remove the coating from the dosage form to permit release of the drug. In general, the coating is on bolt that the polymeric beads or particles and on the exterior of the dosage form, whether it be a pharmaceutical capsule or tablet. Where the dosage form is a tablet, "coating" of the polymeric beads or particles may be obtained by compressing a mixture of particles with the coating material. The term "coating" is thus used herein in a broad sense where the beads or dosage form are substantially surrounded by the "coating" material.

The substances used for the coating are carbohydrates, typically polysaccharides. In the preferred embodiment the polysaccharide is pectin. Polysaccharides useful as a coating are selectively degraded or otherwise solubilized only in the colon but not elsewhere in the digestive tract. Examples are pectin salts, chondroitin, cellulose, hemicellulose, and other sugars that are not degraded by digestive enzymes or otherwise absorbed before reaching the colon.

The amount of polysaccharide coating on the polymer beads or the dosage form will depend on the particular polysaccharide selected, but in any event will be of sufficient thickness to remain intact until reaching the colon. Thus, in the case of the preferred polysaccharide pectin, it has been shown that dissolution and release will depend on the particular pectin selected and primarily its methoxy content. Thus, pectins with a high degree of methoxylation demonstrate a higher degree of protection for the dosage form than those pectins with a lower degree of methoxylation. Pectin USP with a degree of methoxylation of 70% is an example of a preferred material which can be obtained from Bulmer Pectin, UK. The thickness of coating will depend on where it is placed in the dosage form. If the polymer particles themselves are coated, the thickness will be at the thinner end of the range, while a coating on the entire dosage form may be at the higher end of the range. A useful coating thickness range is from about .1 mm to about 1.0 mm.

The following Examples 1 and 2 illustrate the tablet dosage form (Example 1) and the capsule dosage form (Example 2) which may be used in this invention.

### Example 1

### Tablet Configuration

Tablets containing a suitable amount of a corticosteroid entrapped in a MICROSPONGE®¹ System could be prepared with the following general formulation:

| Tablet Component | Weight |
|---|---|
| MICROSPONGE® System with corticosteroid | 250 mg |
| Pectin | 200 mg |
| Dibasic Calcium Phosphate | 100 mg |
| Eudragit 100S² | 100 mg |
| Magnesium Stearate | 10 mg |

| | |
|---|---|
| ²Eudragit 100S - poly(methylmethacrylate-co-methacrylic acid) blocking agent for enteric coating from Röhm Pharma GmbH, Darmstadt, West Germany. | |

¹MICROSPONGE® - Registered trademark of Advanced Polymer Systems, Inc. of Redwood City, California, applied to its cross-linked microsphere polymer beads having an internal porous network.

### Entrapment Preparation

Systems with corticosteroids are:

### A) Hydrocortisone 10% in Acrylates Copolymer

| (Formula per gram of entrapment) | |
|---|---|
| Hydrocortisone | 100 mg |
| Acrylates Copolymer (APS Type E 101³) | 900 mg |

| | |
|---|---|
| ³APS Type E 101, E 140 and E 104 - Polymer beads formed from a copolymer of methyl methacrylate-ethylene glycol dimethacrylate. From Advanced Polymer Systems, Inc., Redwood City, California. The polymers differ by particle size and porosity and all are within the range of about 8-25 microns in diameter. | |

This entrapment can be prepared as described in U.S. Patent No. 5,145,675. Namely, a 5% w/w hydrocortisone solution is prepared by adding 600 mg of hydrocortisone to 12 g of ethanol and then heated to 65°C. Small volumes of this solution (less than 1 ml) are then added to 4.5 g of blank polymer Type E 101 in an amber bottle and then slowly stirred using a spatula for several seconds. This is repeated until a total of 5 g of solution has been added. The bottle is capped and then placed on a roller mill for one hour to mix the contents. The polymer is then dried in an oven at 65°C for 2.5 hours. Due to the low solubility of hydrocortisone in the organic solvents used to prepare the entrapment, to achieve adequate levels of drug in the polymer to make it suitable for therapy, this process is repeated for a second entrapment step with drying of entrapped polymer in the oven at 50°C overnight.

### B) Beclomethasone 5% in Acrvlates Copolymer

| (Formula per gram of entrapment) | |
|---|---|
| Beclomethasone Dipropionate | 50 mg |
| Acrylates Copolymer (APS Type E 140) | 950 mg |

A 2.5% w/w beclomethasone solution is prepared by adding 300 mg of beclomethasone to 12 g of ethanol and then heated to 65°C. Small volumes of this solution (less than 1 ml) are then added to 4.75 g of blank polymer in an amber bottle and then slowly stirred using a spatula for several seconds. This is repeated until a total of 5 g of solution has been added. The bottle is capped and then placed on a roller mill for one hour to mix the contents. The polymer is then dried in an oven at 65°C for 2.5 hours. This process is repeated for the second entrapment step with drying of entrapped microsphere polymer in the oven at 50°C overnight.

### C) Budesonide 5% in Acrylates Copolymer

| (Formula per gram of entrapment) | |
|---|---|
| Budesonide | 50 mg |
| Acrylates Copolymer (APS Type E 104) | 950 mg |

A 2.5% w/w budesonide solution is prepared by adding 300 mg of budesonide to 12 g of tetrahydrofuran and then heated to 65°C. Small volumes of this solution (less than 1 ml) are then added to 4.75 g of blank polymer in an amber bottle and then slowly stirred using a spatula for several seconds. This is repeated until a total of 5 g of solution has been added. The bottle is capped and then placed on a roller mill for one hour to mix the contents. The polymer is then dried in an oven at 65°C overnight. This process is repeated for a second entrapment to obtain the desired payload.

### Tablet Preparation

Tablets are prepared by mixing the indicated amount of polymer entrapment containing the drug and the other ingredients listed in the formulation, except the Eudragit. Tablets are produced by compression compaction using a stainless steel mold and a suitable hydraulic press.

These tablets are then pan-coated with the Eudragit 100S to provide an enteric coating that will allow the tablets to traverse through the stomach without disintegration or premature release of the drug. To coat the tablets, they are placed in a suitably heated rotating drum at about 40-45°C and, while rotating, an appropriate amount of an Eudragit solution in ethanol, isopropanol or acetone is slowly added to the tumbling tablets to obtain a uniform coating by evaporation of the solvent.

### Example 2

| Capsule Configuration | |
|---|---|
| (Formula per Capsule) | |
| MICROSPONGE® System with corticosteroid | 250 mg |
| Pectin (or other suitable polysaccharide) | 150 mg |

Polymer containing the entrapped corticosteroid is prepared as described in Example 1 A), B) or C) above. A pectin solution is prepared by dissolving 1.5 g of pectin (moistened with 0.5 ml of ethanol to facilitate dissolution) in 30 ml of water heated to about 50°C. The suspension is maintained at this temperature with gentle stirring until a clear viscous solution is obtained. Then, 2.5 g of microspheres containing the corticosteroid is placed in a suitable glass or metal container such that it can be rotated while heated and its contents stirred to prevent agglomeration. The pectin solution is then added slowly and in small portions to the polymer, while rotating the vessel and continue heating. As the mixture dries, more pectin solution is added until completion. A granular material, with granules about 0.6-1.0 mm in diameter is obtained. Larger clumps are easily broken into smaller particles with a glass rod or other suitable utensil.

The dry material thus obtained is divided into 400 mg portions and each is placed into a gelatin capsule. Alternatively, if smaller capsules are desired, 200 mg portions can be used.

These capsules, properly sealed, are then placed in a mildly heated coating pan (about 40°C) and, while rotating, an Eudragit 100S solution in ethanol, isopropanol or acetone is slowly added to the tumbling capsules to obtain a uniform coating when the solvent evaporates.

Similar examples of tablet or capsule configurations can be designed by using polymeric entrapments of other drugs like anti-tumor agents, antibacterials, etc., in MICROSPONGE® Systems of different polymer compositions, particle size, and porosities as described in U.S. Patent No. 5,145,675.

The following examples will illustrate the safety of the present dosage form and method.

### Example 3

### In vitro Release of Hydrocortisone

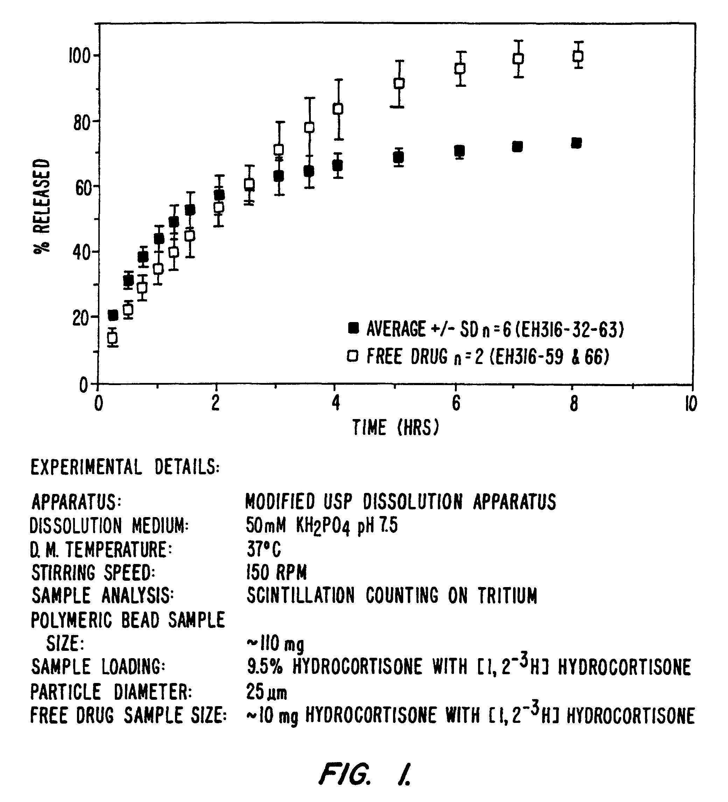

Radiolabelled hydrocortisone was entrapped at a loading of 10% by weight in porous polymeric particles of methyl methacrylate-ethylene glycol dimethacrylate in which the particles had a diameter of about 25 m*µ*. The radioactively labelled hydrocortisone was entrapped in two steps using a 5% ethanolic solution at each step. Release of entrapped hydrocortisone at pH 7.5 was measured using a modified USP dissolution apparatus with a basket of 5 *µ*m mesh and a stirring speed of 150 rpm. Sixty percent of the entrapped hydrocortisone was released in the first two hours with a further 15% released over the next six hours. The dissolution rate of free radioactively labelled hydrocortisone was also measured. The dissolution rate of free hydrocortisone was similar to the entrapped drug for the first two hours but total dissolution occurred in another six hours. The results of this comparative study are shown in Figure 1.

### Example 4

This example demonstrates with animals the safety of the present dosage form when used internally of the body in the G.I. tract. A study was performed in rats in order to determine the time for total elimination of microsponge polymeric beads and the absorption of any extractable materials from the beads in rats after a single oral dose.

Three treatment groups were set up. Twelve rats were weighed and divided into quartiles. Each treatment group was randomly assigned to one rat from each quartile. The first treatment group (Group 1) were controls given 2 ml. of saline by gastric gavage. Group 2 rats were given 2 ml. of a saline slurry containing 200 mg of methyl methacrylate-ethylene glycol dimethacrylate copolymer beads (from Advanced Polymer Systems) radiolabelled with 2.6 million dpm ¹⁴C. Group 3 rats were given a 2 ml saline slurry containing 200 mg of styrene-divinyl benzene copolymer beads (from Advanced Polymer Systems) radiolabelled with 2.6 million dpm ¹⁴C.

Urine and stool were collected from each animal daily on the subsequent 7 days.

After 7 days autopsies were performed on all animals for gross anatomic observation and measurement of radioactivity. The only gross finding was in one animal from group 3 where polymer beads were observed in the pleural space. Radioactivity was found in this material (154,299 dpm/0.048) as well as in the lung (155 dpm/.099). Although not seen on gross exam, radioactivity was found in the lung of one animal from Group 2 (3299 dpm/.066 g) and esophagus (1388 dpm .58 g). These two animals represent technical errors in administering the polymer beads into the stomach. Thus, these animals were excluded from the graphs of the test results.

Radioactivity was measured in the following tissues and fluids for each animal: brain; testes; kidney; liver; spleen; heart; lungs; esophagus; stomach; duodenum; jejunum; ileum; cecum; descending colon; blood; urine; and washing of stool material from the stomach, duodenum, jejunum, ileum, cecum, and descending colon. With the exception of the two animals described above, there was no radioactivity above control in any of the tissues or fluids.

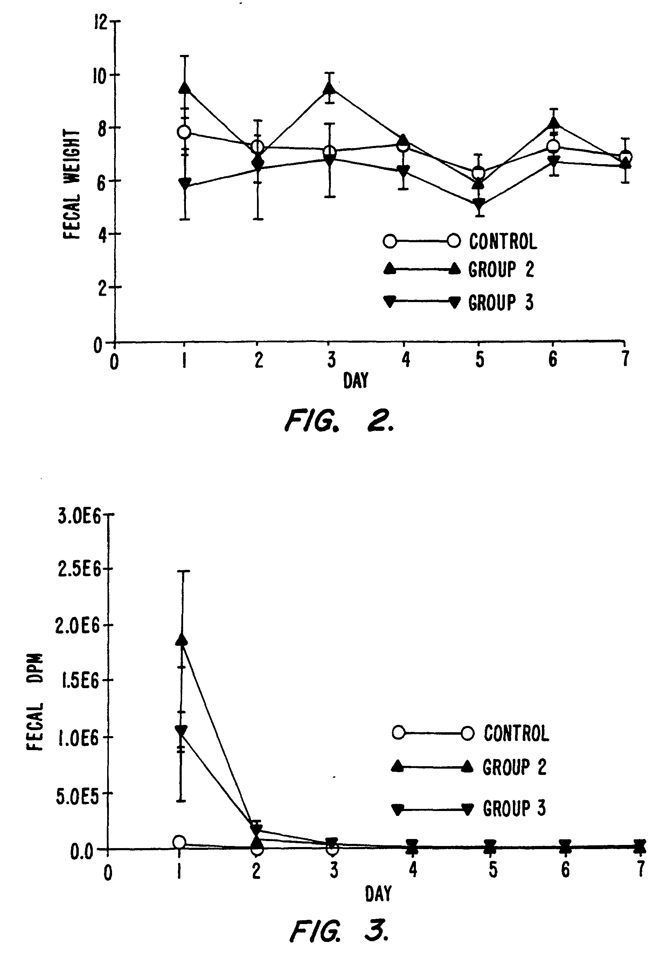

The graph of Figure 2 shows the daily fecal dry weight in each of the three groups of rats. Analysis of variance is sensitive enough to show that fecal weight in the third group is different than the other two groups.

As expected, radioactivity was found in the daily fecal samples in the remaining rats. Figure 3 shows that most of the radioactivity appeared in the feces in the first day. The total calculated recovery of material was 110%, 100%, 95% (Group 2) and 93% 92%, 2% (Group 3).

There were two oddities in the fecal radioactivity measurements. One of the control rats had many counts in one of the fecal samples and one of the Group 3 rats excreted only 2% of radioactivity administered. In this animal there was not radioactivity in the organs or bowel. Thus, the finding was not due to retention of the material. There is no independent reason to reject these samples, and they remain in the reported results.

In conclusion, the data indicate that for both groups receiving radiolabelled microsponges there was rapid and complete elimination of the polymeric beads in the stool.

### Example 5

Another study was conducted to determine the potential toxicity in rats of oral administration for 28 days using porous copolymeric particles formed from methylmethacrylate and dimethyl dimethacrylate made in accordance with Example 6.2 of the '675 patent. The empty particles were fed to rats by themselves and for comparison the same particles were fed in which the particles contained mineral oil in the pores. The conclusion of the study was that feeding of the two forms of the polymeric particles (with and without mineral oil) did not demonstrate any deleterious effect on weight gain, food intake, or fecal output.

### Example 6

### Oral Dose Study of Polymeric Beads in Humans

The study was carried out in five subjects. In brief, each of the subjects collected all of his/her stool for one day before and either seven or nine days after ingesting a dose of ¹⁴C-radiolabelled polymeric microspheres obtained from Advanced Polymer Systems, Inc. The exact dose given was about 1 g containing 10 *µ*Ci of ¹⁴C.

Each daily stool was dried at 60°C for at least one week followed by the determination of ¹⁴C in each stool using a biological oxidizer that incinerated the stool in a stream of N₂ and O₂ at 800°C. The resulting ¹⁴CO₂ was trapped in a scintillation cocktail and ¹⁴C measured using standard liquid scintillation counting technique. The exact amount of ¹⁴C in each stool was calculated using the weights of the samples oxidized, the weight of the total daily stool and the ¹⁴C measured in the oxidized sample.

Values for ¹⁴C in each stool were expressed as a percent of the dose ingested.

The summary shows variability in the timing of excretion. However, it appears that except for subject C.R., all subjects excreted the dose by day six of the study (or five days after ingesting the dose). C.R. was unusual in that she did not have substantial excretion until four days after ingestion of the dose. She hadn't excreted all of her dose by day eight. This slow excretion and the fact that her stool was not collected after day eight accounts for why she only excreted 91.97%of her dose -- a value less than all the other subjects.

The results in the table show that 98.76 ± 1.99 of the ingested doses were excreted in the five subjects. Examination of the values indicates that they are not statistically significantly different from 100%. There was no radioactivity measured in the urine in any of the subjects tests.

In sum, the results demonstrated that ¹⁴C-labelled polymeric microspheres were completely eliminated in the stool after oral ingestion. Furthermore, the peak of radioactivity in feces appeared between 48 and 72 hours after administration, indicating a slow transit through the G.I. tract.

Below in Table I is a summary of the results of the study.

**Table I**

| Summary of Percent of Ingested Microspheres Excreted Per Dav | | | | | | |
|---|---|---|---|---|---|---|
| SUBJECTS | | | | | | Mean ± S.E. |
| | H.J. | C.R. | B.K. | L.N. | D.H. | |
| 1 | 0 | 0 | 0 | 0 | 0 | 0 |
| 2 | 15.24 | 0 | 56.34 | 4.02 | .04 | 23.87 ± 9.57 |
| 3 | 74.55 | .09 | - | 67.95 | 2.51 | 29.02 ± 15.50 |
| 4 | 13.91 | 6.53 | 32.31 | 25.6 | 86.18 | 32.91 ± 12.60 |
| 5 | 0.84 | 70.28 | 10.60 | .015 | 7.53 | 20.43 ± 13.04 |
| 6 | 0.03 | 8.78 | 3.03 | .02 | .87 | 2.55 ± 1.48 |
| 7 | .12 | 2.74 | -.02 | -.01 | .08 | 0.58 ± 0.48 |
| 8 | .08 | 3.55 | .03 | .01 | -.01 | .73 ± 63 |
| 9 | | | | -.02 | -.01 | -.015 |
| 10 | | | | .01 | -.04 | -.015 |
| Total | 104.77 | 91.97 | 102.29 | 97.58 | 97.21 | 98.76 ± 1.99 |

### Example 7

A study involving the gastrointestinal tract in subjects with ileostomy was conducted. The patients were all given a capsule similar to that described in Example 2 wherein the microspheres were loaded with ¹⁴C-hydrocortisone.

In this study, ¹⁴C-hydrocortisone was measured in both the urine and ileal effluent for 3-5 days after ingestion of a capsule containing the test material. In 3 of the subjects, the urine and ileal effluents were collected frequently so that the elimination of ¹⁴C could be timed. The ileal effluents from one subject were accidentally destroyed during the drying process. Thus, only the data from four subjects can be evaluated.

The following are the percentages of ¹⁴C recovered in the ileal effluent and urine for each of the evaluateable subjects. The first table shows recovery for the entire collection, while the second table shows the timing of the excretion in ileal effluent or urine.

| **Entire Collection** | | |
|---|---|---|
| Percentage Recovered | | |
| | Ileal Effluent | Urine |
| Subject 1 | 53.8% | 46.2% |
| Subject 2 | 98.0 | 2.0 |
| Subject 3 | (unable to evaluate) | |
| Subject 4 | 93.3 | 6.7 |
| Subject 5 | 96.3 | 3.7 |
| MEAN | 85.4 | 14.6 |

| **Timed Excretion** | | | | |
|---|---|---|---|---|
| | Percentage Recovered | | | |
| | Ileal Effluent | | Urine | |
| Subject 1 | 0-24 hours | 49.3% | 0-24 hours | 35.0% |
| | 24-48 | 4.3 | 24-48 | 10.9 |
| | 48-72 | 0.3 | 48-72 | 0.4 |
| Subject 2 | 0-14 hours | 0.0% | 0-22 hours | 0.1% |
| | 14-22 | 4.3 | 22-24 | 0.5 |
| | 22-25 | 85.8 | 24-29 | 0.9 |
| | 25-31 | 0.8 | 29-32 | 0.2 |
| | 31-38 | 3.4 | 32-36 | 0.1 |
| | 38-120 | 0.0 | 36-120 | 0.0 |
| Subject 3 | (unable to evaluate) | | (unable to evaluate) | |
| Subject 4 | 0-3 hours | 0.0% | 0-3 hours | 0.1% |
| | 3-9 | 20.5 | 3-9 | 3.1 |
| | 9-16 | 71.5 | 9-16 | 2.4 |
| | 16-25 | 1.2 | 16-25 | 0.9 |
| | 25-96 | 0.0 | 25-32 | 0.2 |
| | | | 32-96 | 0.0 |
| Subject 5 | 0-5 hours | 0.5% | 0-6 hours | 0.0% |
| | 5-16 | 93.5 | 6-10 | 0.3 |
| | | | 10-12 | 0.8 |
| | | | 12-22 | 1.4 |
| | 16-24 | 0.7 | 22-24 | 0.6 |
| | 24-28 | 0.3 | 24-30 | 0.2 |
| | 28-34 | 0.2 | 30-96 | 0.0 |
| | 34-48 | 0.3 | | |
| | 48-72 | 0.6 | | |
| | 72-96 | 0.3 | | |

An examination of the entire collection results indicates that in three of the subjects the ¹⁴C excretion was almost entirely in the ileal effluent. In one subject 53.8% of the ¹⁴C was excreted by way of the ileal effluent. The reason for the difference between the results in this one subject compared to the other three is not known.

An examination of the timed excretion data for the ileal effluents indicates almost all of the excretion occurs within 24 hours after ingestion of the capsule. The exact timing within this 24 hours varied some between the three subjects where such timing could be evaluated. In one, the ileal excretion was greatest at 22-25 hours after ingestion; in another it was at 3-16 hours; and in the third it was 5-16 hours. This variability may be due to differences in residence time for the capsule in the stomach. The transfer of large particles from the stomach to the small intestine depends on many factors. One important factor is the relationship to meals. A capsule such as the one used in this study may not leave the stomach until all nutrients from meals have left the stomach and small intestine.

These results indicate that, in a normal individual (no ileostomy), the active ingedient would be released primarily in the colon, thus maximizing the therapeutic benefits of the drug and minimizing systemic absorption.

## Claims

1. A pharmaceutical composition in unit dosage form suitable for oral ingestion for treating diseases of the colon comprising a plurality of rigid cross-linked polymer beads each defined in a substantially non-collapsible internal pore network and said pores containing entrapped within them a pharmacologically active agent, the beads being coated with a polysaccharide degradable only in the colon and being assembled into the unit dosage form, the unit dosage form being coated with a coating which allows it to pass intact through the gastrointestinal tract until reaching the colon.

2. The pharmaceutical composition of Claim 1 wherein the pharmacologically active agent is a corticosteroid, a non-steroidal anti-inflammatory agent for treatment of inflammatory bowel disease, an anti-tumor agent for treatment of colonic malignancies, an anti-parasitic agent for treatment of parasites or an antibiotic for treatment of infections.

3. The pharmaceutical composition of Claim 1 wherein the unit dosage form is coated with an enteric blocking agent to protect against degradation by acid conditions of the stomach.

4. The pharmaceutical composition of Claim 1 wherein the polysaccharide is pectin.

5. The pharmaceutical composition of Claim 1 wherein the pharmacologically active agent is present in the dosage form in an amount in the range 1-100 mg.

6. The pharmaceutical composition of Claim 1 wherein the dosage form is formulated for treating inflammatory bowel disease and said pharmacologically active agent is a corticosteroid which is hydrocortisone, beclomethasone dipropionate, tixocortol pivalate, dexamethasone, prednisone, budesonide, prednisolone or triamcinolone acetonide.

7. The pharmaceutical composition of Claim 1 wherein the dosage form is a tablet or capsule.

8. The pharmaceutical composition of Claim 1 wherein the polymer beads are formed from a copolymer being styrene-divinylbenzene or methyl methacrylate-ethylene glycol dimethacrylate and the polymer beads have a diameter in the range 5-200 microns.

## Patentansprüche

1. Pharmazeutische Zusammensetzung in einer für die orale Aufnahme geeigneten Dosierungseinheitsform zur Behandlung von Erkrankungen des Colons, umfassend eine Mehrzahl von starren, vemetzten Polymerperlen, die jeweils in einem im wesentlichen nicht-kollabierfähigen inneren Porennetzwerk definiert sind, und die Poren darin eingeschlossen ein pharmakologisch aktives Mittel enthalten, wobei die Perlen mit einem Polysaccharid beschichtet sind, das nur im Colon abbaubar ist, und zu der Dosierungseinheitsform vereinigt sind, wobei die Dosierungseinheitsform mit einem Überzug beschichtet ist, welcher es ihr erlaubt, den gastro-intestinalen Trakt intakt zu passieren, bis sie das Colon erreicht.

2. Pharmazeutische Zusammensetzung nach Anspruch 1, worin das pharmakologisch aktive Mittel ein Corticosteroid, ein nicht-steroidales entzündungshemmendes Mittel zur Behandlung von entzündlicher Darmerkrankung, ein Antitumor-Mittel zur Behandlung von Darmkrebsarten, ein Antiparasitikum zur Behandlung wegen Parasiten oder ein Antibiotikum zur Behandlung von Infektionen ist

3. Pharmazeutische Zusammensetzung nach Anspruch 1, worin die Dosierungseinheitsform zum Schutz gegen Abbau durch die sauren. Bedingungen des Magens mit einem enterischen Blocker beschichtet ist.

4. Pharmazeutische Zusammensetzung nach Anspruch 1, worin das Polysaccharid Pectin ist.

5. Pharmazeutische Zusammensetzung nach Anspruch 1, worin das pharmakologisch aktive Mittel in der Dosierungsform in einer Menge im Bereich von 1 - 100 mg vorliegt

6. Pharmazeutische Zusammensetzung nach Anspruch 1, worin die Dosierungsform zur Behandlung von entzündlicher Darmerkrankung formuliert ist und das pharmakologisch aktive Mittel ein Corticosteroid ist, welches Cordsol, Beclomethasondipropionat, Tixocortolpivalat, Dexamethason, Prednison, Budesonid, Prednisolon oder Triamcinolonacetonid ist.

7. Pharmazeutische Zusammensetzung nach Anspruch 1, worin die Dosierungsform eine Tablette oder Kapsel ist.

8. Pharmazeutische Zusammensetzung nach Anspruch 1, worin die Polymerperlen aus einem Copolymer gebildet sind, das Styrol-Divinylbenzol oder Methylmethacrylat-Ethylenglycoldimethacrylat ist, und die Polymerperlen einen Durchmesser im Bereich von 5 - 200 µm aufweisen.

## Revendications

1. Composition pharmaceutique sous une forme posologique unitaire apte à l'ingestion orale pour le traitement de maladies du côlon, comprenant une pluralité de billes de polymère réticulé rigides chacune définie par un réseau de pores internes substantiellement non aptes à l'affaissement, lesdits pores renfermant à l'état piégé un agent pharmacologiquement actif, les billes étant enrobées d'un polysaccharide dégradable seulement dans le côlon et étant assemblées en la forme posologique unitaire, la forme posologique unitaire étant enrobée avec un enrobage qui permet à cette forme de traverser à l'état intact le tractus gastro-intestinal jusqu'à ce qu'elle atteigne le côlon.

2. Composition pharmaceutique suivant la revendication 1, dans laquelle l'agent pharmacologiquement actif est un corticostéroide, un agent anti-inflammatoire non stéroïdien pour le traitement d'une maladie intestinale inflammatoire, un agent anti-tumoral pour le traitement de maladies malignes du côlon, un agent antiparasitaire pour la destruction de parasites ou un antibiotique pour le traitement d'infections.

3. Composition pharmaceutique suivant la revendication 1, dans laquelle la forme posologique unitaire est enrobée d'un agent de blocage entérique pour la protéger contre la dégradation par les conditions acides de l'estomac.

4. Composition pharmaceutique suivant la revendication 1, dans laquelle le polysaccharide est la pectine.

5. Composition pharmaceutique suivant la revendication 1, dans laquelle l'agent pharmacologiquement actif est présent sous la forme posologique unitaire en une quantité comprise dans l'intervalle de 1 à 100 mg.

6. Composition pharmaceutique suivant la revendication 1, dans laquelle la forme posologique est formulée pour le traitement d'une maladie intestinale inflammatoire et l'agent pharmacologique actif est un corticostéroide qui est l'hydrocortisone, le dipropionate de béclométhasone, le pivalate de tixocortol, la dexaméthasone, la prednisone, le budésonide, la prednisolone ou le triamcinolone acétonide.

7. Composition pharmaceutique suivant la revendication 1, dans laquelle la forme posologique est un comprimé ou une capsule.

8. Composition pharmaceutique suivant la revendication 1, dans laquelle les billes de polymère sont formées d'un copolymère consistant en un copolymère styrène-divinylbenzène ou méthacrylate de méthyldiméthacrylate d'éthylène-glycol et les billes de polymère ont un diamètre compris dans l'intervalle de 5 à 200 micromètres.
